Europäisches Patentamt

European Patent Office

Office européen des brevets

(19)

(11) **EP 0 647 592 B1**

(12) **EUROPÄISCHE PATENTSCHRIFT**

(45) Veröffentlichungstag und Bekanntmachung des Hinweises auf die Patenterteilung:
 **07.05.1997 Patentblatt 1997/19**

(51) Int. Cl.$^6$: **C01C 3/08**, C07C 253/14

(21) Anmeldenummer: **94115315.7**

(22) Anmeldetag: **29.09.1994**

(54) **Verfahren zur Wiedergewinnung von CuCN**

Process for the recovery of CuCN

Procédé pour la récupération de CuCN

(84) Benannte Vertragsstaaten:
 **CH DE FR GB LI**

(30) Priorität: **08.10.1993 DE 4334307**

(43) Veröffentlichungstag der Anmeldung:
 **12.04.1995 Patentblatt 1995/15**

(73) Patentinhaber: **MERCK PATENT GmbH**
 **D-64271 Darmstadt (DE)**

(72) Erfinder:
 • **Heywang, Ulrich, Dr.**
  **D-64289 Darmstadt (DE)**

 • **Weber, Friedel**
  **D-64823 Gross-Umstadt (DE)**

(56) Entgegenhaltungen:
 EP-A- 0 334 188   DE-A- 3 817 914
 GB-A- 2 239 015   US-A- 3 709 921

 • JOURNAL OF ORGANIC CHEMISTRY, Bd.26, Nr.7, 1961 Seiten 2522 - 2524 L FRIEDMAN ET AL. 'Dimethylformamide as a useful solvent in preparing nitriles from aryl halides and cupruos cyanide; improved isolation techniques.'

## Beschreibung

Die Erfindung betrifft ein Verfahren, bei dem das Reagens CuCN aus Reaktionsgemischen wiedergewonnen werden kann, die bei Verfahren zur Herstellung von aromatischen Nitrilen entfallen.

Die Umsetzung von aromatischen halogenierten Verbindungen mit CuCN ist bekannt und wird als Rosenmund-von-Braun-Synthese bezeichnet. Die Umsetzung kann durch folgende Gleichung wiedergegeben werden:

$$ArX + CuCN \rightarrow ArCN + CuX$$

in der $X$ ein Halogenatom bedeutet. Die Umsetzung wird bei einer Temperatur von etwa 150 bis 250 °C und in verschiedenen Lösungsmitteln durchgeführt. Die üblichsten Lösungsmittel umfassen polare aprotische Lösungsmittel wie Dimethylformamid (DMF), Dimethylacetamid (DMA) und N-Methylpyrrolidon (NMP). Normalerweise wird ein Überschuß an CuCN verwendet.

Ein Beispiel eines solchen Verfahrens ist die Herstellung von 4-Methoxy-2-fluorbenzonitril (MFBN) aus 4-Methoxy-2-fluorhalobenzol, die zweckmäßig in DMA oder NMP als Lösungsmittel durchgeführt wird. Nach beendeter Umsetzung wird das Lösungsmittel normalerweise abdestilliert. Das Nitril wird als ein Komplex erhalten, gebunden an das Kupfer(I)-halogenid, das bei der Umsetzung anfällt. Zur Isolierung des Nitrils muß der Komplex zersetzt werden. Verschiedene Verfahren zur Zersetzung sind bekannt. Bei einem dieser Verfahren (US-A-32 59 646) werden konzentrierte Salzsäure und Eisen(III)-chlorid zugegeben und das Produkt wird durch Extraktion mit einem organischen Lösungsmittel isoliert. In einem weiteren Verfahren (L. Friedman und H. Shechter. J. Org. Chem. 1961, Bd. 26, Seiten 2522-2524) wird der Nitrilkomplex mit Äthylendiamin versetzt und das Produkt durch Lösungsmittelextraktion isoliert. In einem weiteren Verfahren der gleichen Literaturstelle wird das Kupfer(I)-halogenid in einer 4 Moläquivalente NaCN enthaltenden wäßrigen Lösung aufgelöst und das Produkt mit einem organischen Lösungsmittel extrahiert.

Die vorgenannten und andere bekannte Verfahren haben erhebliche Nachteile. Das erhaltene Kupferhalogenid wird in schwer wiedergewinnbare Formen umgewandelt, die als solche nicht wiederverwendet werden können. Ferner werden in dem erstgenannten Verfahren sehr giftiger Cyanwasserstoff und Cyan erhalten.

In der DE OS 38 17 914 wird zur Behebung dieser Nachteile vorgeschlagen, das Reaktionsgemisch mit Wasser aufzunehmen, das so erhaltene Gemisch mit einer stöchiometrischen Menge eines Alkalimetallcyanid zu versetzen und die dabei entstehende CuCN-Suspension abzutrennen.

Dieses Verfahren weist folgende Nachteile auf: Zum einen ist es schwierig bei einer Reaktion, von der nicht bekannt ist ob die Umsetzung vollständig war, eine stöchiometrische Menge genau zu dosieren. Dadurch bedingt lassen sich mit diesem Verfahren nur schlecht reproduzierbare Ergebnisse erzielen, so daß eine großtechnische Anwendung nicht möglich ist. Zum anderen entspricht die Reinheit des dabei wiedergewonnen CuCN nicht den Anforderungen zum Einsatz als Reagenz bei der Rosenmund-von-Braun-Synthese.

Die Aufgabe der vorliegenden Erfindung ist es, ein Verfahren zu schaffen, bei dem das teure CuCN-Reagenz in hoher Ausbeute und in akzeptabler Qualität wiedergewonnen werden kann, im Gegensatz zu bisherigen Verfahren, bei denen dieses Reagens praktisch verloren ist oder nur in ungenügender Qualität wiedergewonnen wird.

Die Lösung dieser Aufgabe ergibt sich aus den Patentansprüchen in Verbindung mit der Beschreibung.

Das erfindungsgemäße Verfahren zur Wiedergewinnung von CuCN bei der Rosenmund-von-Braun-Synthese ist durch die folgenden Schritte gekennzeichnet:

a) die Kupfer(I)-halogenide werden von den aromatischen Nitrilen abgetrennt, nachdem die Reaktion der aromatischen halogenierten Verbindung mit Kupfer(I)-cyanid praktisch beendet ist,

b) die Fraktion, welche die Kupfer(I)-halogenide enthält, wird mit einer wäßrigen Lösung eines Alkalimetallcyanides so behandelt, daß eine wäßrige Lösung von Alkalimetalltetracyanocuprat erhalten wird,

c) die so erhaltene Lösung wird gegebenenfalls nach Reinigung, zu einer verdünnten Mineralsäure-Lösung gegeben und

d) das auskristallisierte CuCN wird abgetrennt.

Die Abtrennung erfolgt vorzugsweise durch Zugabe eines apolaren Lösungsmittels zu dem Reaktionsgemisch, wobei die Kupfer(I)-halogenide ausfallen und abgetrennt werden.

Vorzugsweise werden die Schritte a) und b) gleichzeitig durchführt, indem man das Reaktionsgemisch mit einem apolaren Lösungsmittel und einer wäßrigen Lösung eines Alkalimetallcyanides versetzt.

Zur Erzielung einer hohen Reinheit empfiehlt es sich, daß man die in Schritt b) erhaltene Alkalimetalltetracyanocyprat-Lösung mit Aktivkohle behandelt und/oder filtriert.

Vorzugsweise wird die bei Schritt c) entstehende Blausäure durch eine Alkalimetallhydroxid-Lösung geleitet und die entstehende Alkalimetallcyanid-Lösung in Schritt b) wieder eingesetzt.

Bevozugte Ausführungsformen sind weiterhin:

(a) Verfahren, wobei man in Schritt b) 3,5 bis 5,0 mol Alkalimetallcyanid bezogen auf die ursprünglich eingesetzte CuCN verwendet.

(b) Verfahren, wobei man als aromatische halogenierte Verbindung eine aromatische Jod-, Brom- oder Chlorverbindung einsetzt.

(c) Verfahren, wobei man als Alkalimetallcyanid Lithium-, Natrium- oder Kaliumcyanid, vorzugsweise Natriumcyanid einsetzt.

(d) Verfahren, wobei man vor Schritt a) mindestens 60 % des gesamten Lösungsmittels im Reaktionsgemisch entfernt bevor man das apolare Lösungsmittel zusetzt.

(e) Verfahren, wobei man in Schritt a) den verbleibenden Rückstand mit einem Ether versetzt.

(f) Verfahren, wobei man bei gleichzeitiger Durchführung von Schritt a) und b) das Reaktionsgemisch mit Wasser versetzt, ein Alkalimetallcyanid hinzufügt und bei erhöhter Temperatur mit einem organischen Lösungsmittel extrahiert und die Phasen separiert.

Das Alkalimetallcyanid, das in Schritt b) zugesetzt wird, kann zusammen mit dem Wasser vom Schritt a) als Lösung des Alkalimetallcyanids in Wasser, oder als Feststoff oder als eine Lösung der Suspension in einem wässrigen oder nicht-wäßrigen Lösungsmittel dem Reaktionsgemisch zugegeben werden. Gemäß einer bevorzugten Ausführungsform der Erfindung wird vor der Zugabe das Wasser und nach beendeter Umsetzung der aromatischen halogenierten Verbindung mit CuCN das Reaktionsgemisch durch Entfernen eines Teils des Lösungsmittels konzentriert. Durch die Konzentrierung des Gemisches können Kleinere Mengen Wasser im Schritt a) verwendet werden. Dies ermöglicht eine zweckmäßige Wiedergewinnung des Lösungsmittels. Es ist wünschenswert, soviel Lösungsmittel als möglich in diesem Konzentrationsschritt zu entfernen. Es sollte jedoch darauf geachtet werden, daß Temperaturen vermieden werden, die höher sich als das entsprechende Produkt gestattet.

Gemäß einer bevorzugten Ausführungsform des erfindungsgemäßen Verfahrens beträgt die Menge des entfernten Lösungsmittels mindestens 60 % des gesamten Lösungsmittels des Reaktionsgemisches. Es wurde gefunden, daß eine Entfernung von 70 bis 90 % des gesamten Lösungsmittelvolumens in den meisten Fällen vorteilhaft ist. Es kann erforderlich sein, die Temperatur des Reaktionsgemisches vor dem Versetzen mit Wasser einzustellen. Wenn das Verfahren wie üblich bei Atmosphärendruck durchgeführt wird, liegt diese Temperatur normalerweise im Bereich von 30 bis 110 °C, vorzugsweise bei 90 bis 100 °C.

Gemäß einer bevorzugten Ausführungsform der Erfindung ist die aromatische Verbindung eine aromatische Brom- oder Chlorverbindung. Gemäß einer weiteren bevorzugten Ausführungsform ist das Alkalimetallcyanid Natrium oder Kaliumcyanid.

Vorzugsweise wird das Reaktionsgemisch durch Verdampfen des Lösungsmittels konzentriert, insbesondere unter vermindertem Druck. Lösungsmittel, die für die Erfindung besonders geeignet sind, umfassen Dimethylformamid, Dimethylacetamid und N-Methylpyrrolidon. Andere Lösungsmittel können jedoch ebenfalls verwendet werden.

**Beispiel 1**

(a) Herstellung von 4-Methoxy-2-fluorbenzonitril (MFBN)

Eine 700 l Apparatur wird inertisiert und 66,5 kg NMP werden über das Zulaufgefäß eingefüllt.

Unter langsamem Rühren werden 29,8 kg Kupfercyanid durch das Mannloch in die Apparatur eingetragen. Staubbildung und Verschütten ist zu vermeiden. Der Mannlochdeckel wird verschlossen. Im Vakuum bei ca. 120 °C/30 mbar werden etwa 15 kg NMP abdestilliert. Bei 120 °C bis 140 °C in 2 Stunden läßt man 52,5 kg Rohprodukt mit einem Gehalt an 4-Brom-2-Fluoranisol von 42,4 kg zulaufen und noch 20 h bei 140 °C-150 °C rühren. Dann wird bei 30 mbar und 100 °C-120 °C das NMP bis zum noch rührfähigen Rückstand abdestilliert. (Destillat enthält ca. 12% ≙ 6,9 kg MFBN, ≙ 22% d. Th., ≙ 13,1 % v.R., wird dem Folgeansatz zugeschlagen).

In eine 400 l Rührwerksapparatur werden 229 kg Wasser eingefüllt und mit wenig NaOH 32 % auf pH = 10 bis 11 eingestellt. Über das Mannloch werden 53,9 kg Natriumcyanid eingetragen und bei 70 °C-75 °C bis zur blanken Lösung gerührt.

Ist das Natriumcyanid gelöst läßt man die Lösung bei 90 °C Sumpftemperatur in die 700 l-Apparatur langsam einlaufen. Es wird auf 50 °C-60 °C abgekühlt und über das Zulaufgefäß 100 kg Toluol zugegeben. Nach der Toluolzugabe wird noch 30 Minuten nachgerührt. Man läßt 30 Minuten absitzen und trennt dann die Wasserphase ab. Die Toluolphase wird abgelassen. Die Wasserphase wird noch 3 mal mit jeweils 30 kg Toluol nachextrahiert. Alle abgetrennten Toluolphasen werden gesammelt, in die 700-l-Apparatur eingefüllt und mit 50 kg Wasser ausgerührt. Das abgetrennte Wasser wird mit der 1. Wasserphase vereinigt und bis zur Freisetzung des Kupfercyanids eingelagert.

Die in der Apparatur verbliebene Toluolphase wird bei Normaldruck andestilliert, bis das Destillat wasserfrei abläuft. Es wird auf 40 °C-50 °C abgekühlt, 0,5 kg Aktivkohle zugegeben und 30 Minuten gerührt. Man filtriert über einen Seitzfilter ab in eine Palette. Apparatur und Filter werden mit 20 kg Toluol nachgespült.

Das Filtrat wird auf ca. 60 kg im Vakuum bei ca. 30 mbar eingedampft. Der Destillations-Rückstand wird auf -15 °C eingekühlt, bei dieser Temperatur über Nacht gerührt und abgeschleudert. Es wird mit 2 × 2,5 kg kaltem Toluol (-15 °C) nachgewaschen. Die erhaltenen Kristalle werden über Nacht im Vakuum bei 40 °C im Trockenschrank getrocknet. Man erhält 15,7 kg 4-Cyano-3-fluoranisol. Das sind 50,2 % d. Th. oder 29,9 % vom Rohstoff. Ausbeute im Folgeansatz : 22,6 kg, das sind 72,2 % d. Th. oder 43,0 % vom Rohstoff.

b) Freisetzung von Kupfercyanid:

Die Wasserphase aus Stufe a) wird in die 700-l-Apparatur gefüllt, mit 0,5 kg Aktivkohle versetzt und bei Normaldruck bis 100 °C Kopftemperatur zur Entfernung von Toluolspuren andestilliert.

Vom Destillat wird die Unterphase abgetrennt und abgelassen. Sobald die Kopftemperatur erreicht ist, wird über das Seitzfilter in eine Palette abfiltriert und mit 20 kg Wasser Apparatur und Filter nachgespült. Es werden ca. 290 kg-300 kg Filtrat (Cyanocupratlösung) erhalten.

In einer 400 l Apparatur werden über das Zulaufgefäß 126 kg Natronlauge 32 % und 74 kg VE-Wasser eingefüllt. Ins Zulaufgefäß werden weitere 50 kg Natronlauge für eine evtl. pH-Nachregelung eingefüllt.

In eine 700-l-Apparatur werden 260 kg Wasser eingefüllt. Über das Zulaufgefäß läßt man 65,0 kg Schwefelsäure konz. zulaufen und spült mit 40 kg VE-Wasser nach.

Das Filtrat (Cyanocupratlösung) wird über eine Dosierpumpe in 2-3 h zudosiert. Die freiwerdende Blausäure wird in der 400-l-Apparatur absorbiert und als NaCN-Lösung im Folgeansatz eingesetzt. Der Ansatz wird zum Rückfluß erwärmt und 30 Minuten gerührt. Man kühlt auf 10 °C ab und nutscht über die Drucknutsche. Danach wird mit 80 kg Wasser, dem 0,5 kg Natronlauge 32 % zugesetzt sind, nachgewaschen. Dann wird noch 2 × mit 40 kg Wasser nachgewaschen. Die anfallenden Filtrate werden gesammelt, und bis zur späteren Entsorgung der Mutterlauge eingelagert.

Die abgetrennten Kriställe werden über Nacht in der Drucknutsche bei 100 °C im Vakuum unter leichtem $N_2$-Strom getrocknet.

Ausbeute:

Pro Ansatz erhält man so 29,5 kg Kupfercyanid mit einem Gehalt >99 %
29,5 kg Kupfercyanid = 99 % v.R CuCN

**Beispiel 2**

(a) Herstellung der Tetracyanocupratlösung
Arbeitsschritte

In einer 400-l-Rührwerksapparatur werden 214 kg Wasser vorgelegt. Unter langsamem Rühren werden 50,3 kg Natriumcyanid durch das Mannloch in die Appartur eingetragen. Bei 40-50 °C wird gerührt, bis das NaCN gelöst ist.

In eine 700-l-Rührwerksapparatur werden 80 kg Wasser vorgelegt.

Über das offene Mannloch werden unter Rühren bei minimaler Drehzahl 49 kg Kupferrückstände aus der Nitrilsynthese eingetragen. Das Mannloch wird verschlossen und der Ansatz auf 60 °C-70 °C erwärmt.

Sind 60 °C erreicht, läßt man langsam die NaCN-Lösung aus der 400-l-Apparatur zulaufen. Dabei löst sich das Kupfersalz auf. Es wird weiter zum Rückfluß erwärmt und 50 kg Wasser/MTB-Ether/Ammoniak abdestilliert. Dann kühlt man auf 90-95 °C ab, entnimmt eine Probe unter Rühren und kontrolliert auf vollständige Lösung des Cu-Salzes.

0,5 kg Aktivkohle werden in 5 kg Wasser angerührt und über das Mannloch zugegeben. Es wird noch 30 Minuten bei gleicher Temperatur weitergerührt und dann über das Seitzfilter heiß abfiltriert. Anschließend wird das Filtrat ausgewogen und für die folgende HCN-Abspaltung beiseite gestellt.

(b) Freisetzung von Kupfercyanid:

In eine Apparatur ① werden über das Zulaufgefäß 118 kg Natronlauge 32 % und 70 kg Wasser eingefüllt. Ins Zulaufgefäß werden weitere 50 kg Natronlauge für die evtl. pH-Nachregelung eingefüllt.

In eine Apparatur ② füllt man 150 kg Wasser. Über das Zulaufgefäß. läßt man 75,6 kg Schwefelsäure konz. zulaufen und erwärmt auf 90-95 °C. Rührwerk auf volle Drehzahl einregeln. Filtrat aus Stufe (a) (Cyanocupratlösung) über Dosierpumpe in 2-3 h zudosieren. Dosiermenge: 2-2,5 kg/min. Die freiwerdende Blausäure wird in Apparatur ① absorbiert. Der Ansatz wird zum Rückfluß erwärmt und 30 Minuten gerührt. Man kühlt auf 10 °C ab und nutscht über eine Drucknutsche ab.

Die Mutterlauge wird aufgefangen. Die anfallenden Filtrate werden gesammelt, und bis zur späteren Entsorgung der Mutterlauge, eingelagert. Die abgetrennten Kristalle werden über Nacht in der Drucknutsche bei 100 °C im Vakuum unter leichtem $N_2$-Strom getrocknet.

Ausbeute:

Pro Ansatz erhält man so 28,0 kg Kupfercyanid mit einem Gehalt >99 %
28,0 kg Kupfercyanid = 57,1 % v.R.

**Beispiel 3**

130 ml NMP, 50 g 4-Acetoxy-4'-brom-biphenyl und 25 g Kupfercyanid werden vereinigt und im Vakuum (ca. 30 mbar) bei einer Kopftemperatur von 130 bis 135 °C ca. 15 ml NMP abdestilliert. Anschließend wird der Ansatz bei Normaldruck bei einer Badtemperatur von 185 °C 5 Stunden gehalten. 54,7 g Natriumcyanid werden in 235 ml Wasser gelöst.

Nach 5 Stunden wird bei einer Badtemperatur von ca. 185 °C vorsichtig Vakuum angelegt und das NMP weitgehendst abdestilliert. Der Sumpf wird auf etwa 130 °C abgekühlt. Bei der Zugabe von Toluol wird der Rückstand erst bei etwa 50 ml wieder halbwegs rührbar. Insgesamt werden 160 ml Toluol zugegeben. Der Ansatz wird auf eine Sumpftemperatur von 5 °C abgekühlt und die Natriumcyanidlösung zugetropft. Bereits nach ca. 30 ml ist die Sumpftemperatur wieder auf 25 °C angestiegen. Der Rest der Lösung wird so zugetropft, daß 25 °C nicht überschritten wurden Nach beendeter Zugabe ist ein Feststoff ausgefallen. Dieser wurde abgenutscht, mit Wasser und Toluol gewaschen und im Vakuum über Nacht bei 75 °C getrocknet. Die Toluolphase des Filtrats wird abgetrennt, mit Wasser gewaschen und einrotiert. Die wäßrige Cyanidphase wird analog Beispiel 2(b) der Kupfercyanidrecyclisierung zugeführt. Aus 125 ml Toluol unter Zusatz von 1 g Aktivkohle erhält man 32 g 4-Cyano-4'-hydroxybiphenyl, Schmp. 109-111 °C (78,5 % d.Th.).

## Beispiel 4

Herstellung von p-Methoxybenzonitril

92,5 g (0,5 mol) 4-Bromanisol, 175 ml Dimethylacetamid und 58,2 g (0,65 mol) CuCN werden 6 Stunden unter Rückfluß erhitzt. Anschließend werden unter vermindertem Druck ca. 150 ml Dimethylacetamid abdestilliert. Der Destillationsrückstand wird auf 100 °C abgekühlt und mit 500 ml Toluol versetzt. Dazu läßt man die Lösung von 127,4 g (2,6 mol) Natriumcyanid in 550 ml Wasser zutropfen. Die Toluolphase wird abgetrennt und zum Rückstand eingeengt. Dieser Rückstand wird aus Ethanol umkristallisiert. Man erhält 50 g (75 % Ausbeute) p-Methoxybenzonitril vom Fp. 61-62 °C und einer GC-Reinheit >99 %.

Die wäßrige Phase wird kurz andestilliert, mit Aktivkohle behandelt und wie in Beispiel 2 (b) beschrieben in Schwefelsäure zersetzt. Man erhält so 57,7 g (99 % des Ausgangsmaterials) CuCN zurück.

## Vergleichsbeispiel 1 (gemäß DE 38 17 914)

Versuch zur Herstellung von Kupfercyanid aus NMP-Lösung durch Zugabe von Kalium-Cyanid-Lösung:

1282 g feuchte (entspricht 1000 g trockene) Kupferrückstände (Kupfergehalt: ca. 8 mol) werden in 1470 ml NMP gemeinsam mit 49 g Aktivkohle und 49 g Celite 2 H bei 140 gerührt. Dabei destillieren etwa 312 g Destillat ab. Anschließend wird die Lösung bei 115 °C blankfiltriert und mit 740 ml NMP nachgewaschen. Auf dem Filter bleibt ein Rückstand von, 395 g zurück. Zu dem Filtrat wird die Lösung von 300 g Kaliumcyanid in 568 g Wasser gegeben und 1 h bei 100 °C am Rückfluß gekocht. Das ausgefallene Kupfercyanid wird abgesaugt und getrocknet. Ausbeute: 520 g, theoretisch erwartet ca. 400 g (5 mol). Aus der Analyse ergibt sich, daß das Produkt etwa die Formel KCu(CN)$_2$ hat.

## Vergleichsbeispiel 2 (gemäß DE 38 17914)

Versuch zur Herstellung von Kupfercyanid aus NMP-Lösung durch Zugabe von Natriumcyanid-Lösung:

Analog Vergleichsbeispiel 1, jedoch mit 225 g Natriumcyanid. Es fiel jedoch kein Produkt aus.

## Patentansprüche

1. Verfahren zur Wiedergewinnung von CuCN aus Reaktionsgemischen von aromatischen Nitrilen und Kupfer-(I)-halogeniden, dadurch gekennzeichnet, daß man

a) die Kupfer(I)-halogenide von den aromatischen Nitrilen abtrennt, nachdem die Reaktion der aromatischen halogenierten Verbindung mit Kupfer-(I)-cyanid praktisch beendet ist,

b) die Kupfer-(I)-halogenide enthaltende Fraktion mit einer wäßrigen Lösung einer 3,5 bis 5-fachen molaren Menge Alkalimetallcyanid, bezogen auf die ursprünglich eingesetzte Menge CuCN versetzt, wodurch eine wäßrige Lösung von Alkalimetalltetracyanocuprat erhalten wird,

c) die wäßrige Lösung, gegebenenfalls nach Reinigung, zu einer verdünnten Mineralsäure-Lösung gibt, und

d) das auskristallisierte CuCN abtrennt,

wobei entweder das Reaktionsgemisch selbst vor der Verfahrensstufe a) nach Abdestillieren von bis zu 90 % des Lösungsmittels bder die durch die Verfahrensstufe b) erhaltene wäßrige Lösung mit einem apolaren Lösungsmittel versetzt wird.

2. Verfahren nach Anspruch 1, dadurch gekennzeichnet, daß man in Schritt a) ein apolares Lösungsmittel zu dem Reaktionsgemisch gibt, wobei die Kupfer(I)-halogenide ausfallen und abgetrennt werden.

3. Verfahren nach Anspruch 1, dadurch gekennzeichnet, daß man die Schritte a) und b) gleichzeitig durchführt, indem man das Reaktionsgemisch mit einem apolaren Lösungsmittel und einer wäßrigen Lösung eines Alkalimetallcyanides versetzt.

4. Verfahren nach einem der vorangegangenen Ansprüche, dadurch gekennzeichnet, daß man die in Schritt b) erhaltene Alkalimetalltetracyanocuprat-Lösung mit Aktivkohle behandelt und/oder filtriert.

**5.** Verfahren nach einem der vorhergehenden Ansprüche, dadurch gekennzeichnet, daß man die bei Schritt c) entstehende Blausäure durch eine Alkalimetallhydroxid-Lösung leitet und die entstehende Alkalimetallcyanid-Lösung in Schritt b) wieder einsetzt.

**6.** Verfahren nach einem der vorhergehenden Ansprüche, dadurch gekennzeichnet, daß man als aromatische halogenierte Verbindung eine aromatische Jod-, Brom- oder Chlorverbindung einsetzt.

**7.** Verfahren nach einem der vorangehenden Ansprüche, dadurch gekennzeichnet, daß man in Schritt b) als Alkalimetallcyanid Lithium-, Natrium- oder Kaliumcyanid, vorzugsweise Natriumcyanid einsetzt.

**8.** Verfahren nach Anspruch 2, dadurch gekennzeichnet, daß man mindestens 60 % des gesamten Lösungsmittels im Reaktionsgemisch entfernt, bevor man das apolare Lösungsmittel zusetzt.

**9.** Verfahren nach Anspruch 8, dadurch gekennzeichnet, daß man den verbleibenden Rückstand mit einem Ether versetzt.

**10.** Verfahren nach Anspruch 3, dadurch gekennzeichnet, daß man das Reaktionsgemisch mit Wasser versetzt, ein Alkalimetallcyanid hinzufügt und bei erhöhter Temperatur mit einem organischen Lösungsmittel extrahiert und die Phasen separiert.

**Claims**

**1.** Process for the recovery of CuCN from reaction mixtures of aromatic nitriles and copper (I) halides, characterized in that

a) the copper (I) halides are separated off from the aromatic nitriles after the reaction of the aromatic halogenated compound with copper(I) cyanide is virtually complete,
b) the fraction containing the copper(I) halides is admixed with an aqueous solution of 3.5 to 5 times the molar amount of alkali metal cyanide, based on the amount of CuCN originally used, which gives an aqueous solution of alkali metal tetracyanocuprate,
c) the aqueous solution, if appropriate after purification, is added to a dilute mineral acid solution, and
d) the CuCN crystallized out is separated off,

either the reaction mixture itself prior to the process stage a), after distilling off up to 90% of solvent, or the aqueous solution obtained by process stage b), being admixed with an apolar solvent.

**2.** Process according to Claim 1, characterized in that an apolar solvent is added to the reaction mixture in step a), the copper(I) halides precipitating out and being separated off.

**3.** Process according to Claim 1, characterized in that the steps a) and b) are carried out simultaneously by admixing the reaction mixture with an apolar solvent and an aqueous solution of an alkali metal cyanide.

**4.** Process according to one of the preceding claims, characterized in that the alkali metal tetracyanocuprate solution obtained in step b) is treated with activated carbon and/or filtered.

**5.** Process according to one of the preceding claims, characterized in that the hydrocyanic acid formed in step c) is passed through an alkali metal hydroxide solution and the resulting alkali metal cyanide solution is reused in step b).

**6.** Process according to one of the preceding claims, characterized in that the aromatic halogenated compound used is an aromatic iodine compound, bromine compound or chlorine compound.

**7.** Process according to one of the preceding claims, characterized in that the alkali metal cyanide used in step b) is lithium cyanide, sodium cyanide or potassium cyanide, preferably sodium cyanide.

**8.** Process according to Claim 2, characterized in that at least 60% of all of the solvent in the reaction mixture is removed before the apolar solvent is added.

**9.** Process according to Claim 8, characterized in that the remaining residue is admixed with an ether.

**10.** Process according to Claim 3, characterized in that the reaction mixture is admixed with water, an alkali metal cyanide is added and the mixture is extracted with an organic solvent at elevated temperature and the phases are separated.

**Revendications**

**1.** Procédé pour la récupération de CuCN à partir de mélanges réactionnels de nitriles aromatiques et d'halogénures cuivreux, caractérisé en ce que

a) on sépare les halogénures cuivreux des nitriles aromatiques, des que la réaction du composé aromatique halogéné avec le cyanure cuivreux est pratiquement terminée,
b) on ajoute à la fraction contenant des halogénures cuivreux une solution aqueuse d'une quantité 3,5 à 5 fois molaire d'un cyanure de métal alcalin, par rapport à la quantité de CuCN initialement utilisée, pour obtenir une solution aqueuse de tétracyanocuprate de métal alcalin,

c) on ajoute la solution aqueuse, éventuellement après purification, à une solution diluée d'un acide minéral, et

d) on sépare le CuCN cristallisé,

en ajoutant un solvant non polaire soit au mélange réactionnel lui-même, avant l'étape de processus a), après élimination par distillation de jusqu'à 90 % du solvant, soit à la solution aqueuse obtenue par l'étape de processus b).

2. Procédé selon la revendication 1, caractérisé en ce que, dans l'étape a), on ajoute un solvant non polaire au mélange réactionnel, ce par quoi les halogénures cuivreux précipitent et sont séparés.

3. Procédé selon la revendication 1, caractérisé en ce que l'on effectue simultanément les étapes a) et b), en ajoutant au mélange réactionnel un solvant non polaire et une solution aqueuse d'un cyanure de métal alcalin.

4. Procédé selon l'une des revendications précédentes, caractérisé en ce que l'on traite par du charbon actif et/ou filtre la solution de tétracyanocuprate de métal alcalin, obtenue dans l'étape b).

5. Procédé selon l'une des revendications précédentes, caractérisé en ce que l'on fait passer l'acide cyanhydrique, formé dans l'étape c), à travers une solution d'hydroxyde de métal alcalin, et la solution résultante de cyanure de métal alcalin est réutilisée dans l'étape b).

6. Procédé selon l'une des revendications précédentes, caractérisé en ce que l'on utilise comme composé aromatique halogéné un composé aromatique iodé, bromé ou chloré.

7. Procédé selon l'une des revendications précédentes, caractérisé en ce que, dans l'étape b), on utilise comme cyanure de métal alcalin le cyanure de lithium, de sodium ou de potassium, de préférence le cyanure de sodium.

8. Procédé selon la revendication 2, caractérisé en ce que, avant d'ajouter le solvant non polaire, on élimine au moins 60 % du solvant total présent dans le mélange réactionnel.

9. Procédé selon la revendication 8, caractérisé en ce que l'on ajoute un éther au résidu obtenu.

10. Procédé selon la revendication 3, caractérisé en ce que l'on ajoute de l'eau au mélange réactionnel, on y ajoute un cyanure de métal alcalin, puis on l'extrait à haute température avec un solvant organique et on sépare les phases.